# EUROPEAN PATENT SPECIFICATION

(11) **EP 1 019 015 B1**
(45) Date of publication and mention of the grant of the patent: **05.02.2003**
(21) Application number: 98910060.7
(22) Date of filing: 27.02.1998
(51) Int. Cl.: A61K 7/16

(54) **ORAL COMPOSITION EXHIBITING IMPROVED ANTIBACTERIAL UPTAKE AND RETENTION**
ORALE ZUSAMMENSETZUNG MIT VERBESSERTER ANTIBAKTERIELLER AUFNAHME UND ZURÜCKHALTUNG
COMPOSITION ORALE PRESENTANT UNE ABSORPTION ET UNE RETENTION ANTIBACTERIENNE AMELIOREE

(30) Priority: 28.02.1997 US 808607
(43) Date of publication of application: 19.07.2000
(73) Proprietor: Colgate-Palmolive Company, New York, N.Y. 10022 (US)
(72) Inventor: NABI, Nuran, Cranbury, NJ 08512 (US); GAFFAR, Abdul, Princeton, NJ 08902 (US)
(74) Representative: UEXKÜLL & STOLBERG
(86) International application number: US9803705
(87) International publication number: WO98037860

(56) References cited:
- EP-A- 0 371 542
- EP-A- 0 750 901
- US-A- 4 022 880

## Description

### Background Of The Invention

### 1. Field of the Invention

The present invention relates to oral care compositions which are designed to improve the effectiveness of antibacterial compounds in the retardation and prevention of bacterial plaque accumulation on the teeth.

### 2. The Prior Art

Dental plaque is a soft deposit which forms on teeth and is comprised of an accumulation of bacteria and bacterial by-products. Plaque adheres tenaciously at the points of irregularity or discontinuity, e.g., on rough calculus surfaces, at the gum line and the like. Besides being unsightly, plaque is implicated in the occurrence of gingivitis and other forms of periodontal disease.

A wide variety of antibacterial agents have been suggested in the art to retard plaque formation and the oral infections and dental disease associated with plaque formation. For example, halogenated hydroxydiphenyl ether compounds such as triclosan are well known to the art for their antibacterial activity and have been used in oral compositions to counter plaque formation by bacterial accumulation in the oral cavity.

Although antibacterial agents such as triclosan are highly effective in killing bacteria which are responsible for plaque formation, it is difficult to maintain an effective level of such agents on dental tissue for a significant time period after their application. Thus, once applied, it is important that the antibacterial compound be maintained in continuing adherence to the teeth and adjacent oral gingival mucosa thereby retarding washout of the antibacterial compound from infected areas of dental tissue by saliva antibacterial compound from infected areas of dental tissue by saliva present in the mouth. This allows for a sufficient amount of compound to remain in contact with the dental tissue and achieve a protracted and therefore enhanced antibacterial effect.

EP-A-0 750 901 discloses an antibacterial oral composition containing an antibacterial compound such as Triclosan and a monoalkyl phosphate compound whereby the uptake and retention of the antibacterial compound on dental tissue is substantially increased due to the presence of the monoalkyl phosphate compound.

### Summary Of The Invention

Disclosed in U.S. 5,605,676 is the discovery that the presence of relatively low concentrations, e.g., 0.1 to 5% by weight of a monoalkyl phosphate compound in an oral composition containing an antibacterial halogenated diphenyl ether or phenolic compound substantially increases uptake and retention of the antibacterial compound on dental tissue. Further experimentation, including a human clinical study, demonstrates that when an anionic surfactant is present in the oral composition and the weight ratio of the anionic surfactant to monoalkyl phosphate ranges from 2:1 to 1:2, the antibacterial efficacy of the composition is materially enhanced.

### Detailed Description Of The Preferred Embodiments

The term "oral composition" is used herein to designate products which, in the ordinary course of usage, are retained in the oral cavity for a time sufficient to contact substantially all of the dental surfaces but are not intentionally ingested. Such products include, for example, dentifrices, gels, mouthwashes, chewing gums and lozenges.

Halogenated diphenyl ether antibacterial compounds for use in the oral care compositions of the present invention particularly desirable from considerations of antiplaque effectiveness and safety include 2,4,4'-trichloro-2'-hydroxy-diphenyl ether (triclosan) and 2,2'-dihydroxy-5,5'-dibromodiphenyl ether.

Phenolic compounds useful in the practice of the present invention include phenol and its homologs, mono and polyalkyl and aromatic halophenols, resorcinol and its derivatives and bisphenolic compounds, such phenolic compounds being more fully disclosed in U.S. 5,368,844. Preferred phenolic compounds are n-hexyl resorcinol and 2,2'-methylene bis (4-chloro-6-bromophenol).

The halogenated diphenyl ether or phenolic antibacterial compound is present in the oral composition of the present invention in an effective antiplaque amount, typically 0.05%-2.0% by weight, and preferably 0.1%-1% by weight of the oral composition.

The term "monoalkyl phosphate compound" includes within its meaning a monoalkyl or monoalkenyl phosphate, mixtures thereof, and a salt thereof having the formula 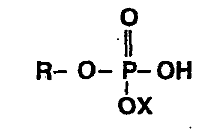 wherein R is an alkyl or alkenyl group having 6 to 18 carbon atoms and X is hydrogen, sodium, potassium or ammonium.

Monoalkyl phosphate compounds useful in the practice of the present invention include monolauryl phosphate, monooctyl phosphate, sodium lauryl phosphate and sodium monooctyl phosphate.

Monoalkyl phosphate compounds are known to the art and are used in combination with dialkyl phosphates in skin care compositions, e.g., U.S. Patent No. 4,139,485 and as surfactants (U.S. Patent No. 4,139,485, U.S. Patent No. 4, 152, 421 and U.S. Patent No. 4,350,680). Combinations of mono- and dialkyl phosphate salt have been disclosed as having efficacy as anticaries agents (U.S. Patent No. 5,019,373). Japanese Patent 6271440 discloses an antimicrobial compound of the formula Am+ Xm- wherein Am+ is a nitrogen containing antimicrobial agent and X- is an 8 to 20 carbon monoalkyl or monoalkenyl phosphoric acid ion and m is the valence of cation A. There is no suggestion in the prior art that monoalkyl phosphate compounds particularly when used in combination with an anionic surfactant and an antibacterial compound would have enhanced antibacterial effect.

Examples of anionic surfactants suitable for use in the practice of the present invention include water-soluble salts of higher fatty acid monoglyceride monosulfates, such as the sodium salt of the monosulfated monoglyceride of hydrogenated coconut oil fatty acids, higher alkyl sulfates, such as sodium lauryl sulfate, alkyl aryl sulfonates, such as sodium dodecyl benzene sulfonate, higher alkyl sulfoacetates, higher fatty acid esters of 1,2-dihydroxypropane sulfonate, and the substantially saturated higher aliphatic acyl amides of lower aliphatic amino carboxylic acid compounds, such as those having 12 to 16 carbons in the fatty acid, alkyl or acyl radicals, and the like. Examples of the last mentioned amides are N-lauroyl sarcosine, and the sodium, potassium, and ethanolamine salts of N-lauroyl, N-myristoyl, or N-palmitoyl sarcosine which should be substantially free from soap or similar higher fatty acid material. A preferred anionic surfactant is sodium lauryl sulfate.

The combination of a monoalkyl phosphate compound and an anionic surfactant, free of any dialkyl phosphate compound is found to significantly enhance the antibacterial effect of halogenated diphenyl ether and phenolic antibacterial compounds on dental tissue when the monoalkyl phosphate and the anionic surfactant are incorporated in the oral composition in amounts effective to achieve such enhancement, such amounts being within the range of 0.1 to 5% of each ingredient by weight within the oral composition, preferably 0.2% to 3%, and most preferably 0.3% to 1.2% by weight of each ingredient within the oral composition, the weight ratio of anionic surfactant to monoalkyl phosphate ranging from 2:1 to 1:2.

In the preparation of an oral care composition in accordance with the practice of the present invention, an orally acceptable vehicle including a water-phase with humectant is present. The humectant is preferably glycerine, sorbitol, and/or propylene glycol. Water is present typically in amount of at least 10% by weight, generally 25 to 70% by weight and the humectant concentration typically totals 10-80% by weight of the oral composition.

Dentifrice compositions also typically contain polishing materials including crystalline silica, having a particle size of up to about 20 microns, such as available as Zeodent 115, silica gel or colloidal silica, complex amorphous alkali metal aluminosilicates, as well as sodium bicarbonate, calcium carbonate, calcium pyrophosphate, dicalcium phosphate and alumina.

Dentifrices prepared in accordance with the present invention typically contain a natural or synthetic thickener in proportions of 0.1 to 5% by weight, preferably 0.5 to 2% by weight. Suitable thickeners include Irish moss, i-carrageenan, gum tragacanth, starch, polyvinylpyrrolidone, hydroxyethylpropyl cellulose, hydroxybutyl methyl cellulose, hydroxypropyl methylcellulose, hydroxyethyl cellulose sodium carboxymethyl cellulose (NaCMC), and colloidal silica such as those available as finely ground Syloid 244 and Sylodent 15.

The oral composition may also contain a source of fluoride ions, or fluoride-providing compound, as an anti-caries agent, in an amount sufficient to supply 25 ppm to 5,000 ppm of fluoride ions and preferably 500 to 1500 ppm fluoride ions. Among these compounds are inorganic fluoride salts, such as soluble alkali metal salts, for example, sodium fluoride, potassium fluoride, sodium fluorosilicate, ammonium fluorosilicate and sodium monofluorophosphate, as well as tin fluorides, such as stannous fluoride and stannous chloride.

Any suitable flavoring or sweetening material may also be employed. Examples of suitable flavoring constituents include flavoring oils, e.g. oil of spearmint, peppermint, wintergreen, clove, sage, eucalyptus, marjoram, cinnamon, lemon, and orange, and methyl salicylate. Suitable sweetening agents include sucrose, lactose, maltose, xylitol, sodium cyclamate, perillartine, AMP (aspartyl phenyl alanine methyl ester), saccharine and the like. Suitably, flavor and sweetening agents may each or together comprise from 0.1% to 5% more of the oral care composition. Moreover, flavor oil is believed to aid the dissolving of water insoluble non-cationic halogenated diphenyl ether antibacterial agents such as triclosan.

Various other materials may be incorporated in the oral preparations of this invention such as whitening agents, including urea peroxide, calcium peroxide, and hydrogen peroxide, preservatives, vitamins such as vitamin B6. B12, E and K, silicones, chlorophyll compounds, potassium salts for the treatment of dental hypersensitivity such as potassium nitrate as well as antitartar agents such as sodium tripolyphosphate and di- and tetraalkali metal pyrophosphate salts such as di- and tetrasodium pyrophosphate. These agents, when present, are incorporated in the compositions of the present invention in amounts which do not substantially adversely affect the properties and characteristics desired.

The preparation of dentifrices is well known in the art. U.S. Patent Nos. 3,996,863, 3,980,767, 4,328,205, and 4,358,437 describe toothpastes and methods of production thereof, which may be utilized for production of the dentifrices according to the present invention. Further, discussion of the preparation of oral compositions is presented in Harry's Cosmeticology, Seventh Edition, 1982, edited by J.B. Wilkinson and R.J. Moore, published by Chemical Publishing of New York, pages 609 to 617.

All amounts and proportions referred to herein and in the appended claims are by weight, unless otherwise indicated.

### Example I

A liquid dentifrice of the present invention designated Composition A was prepared following the above discussed procedure containing 1.0% by weight sodium lauryl phosphate and 0.5% by weight sodium lauryl sulfate, an anionic surfactant. the detailed formulation for which is shown in Table I. This composition was repeated a series of times as Compositions B, C, D, E, and F; these compositions differing from Composition A only in the relative quantities of sodium lauryl phosphate and sodium lauryl sulfate within each composition (q.s. water), as shown in Table II.

**TABLE I**

| **Liquid Dentifrice Formulation "A"** | |
|---|---|
| ***Ingredients*** | ***Wt.%*** |
| Sorbitol | 20.00 |
| Glycerol | 20.00 |
| Propylene Glycol | 0.50 |
| Sodium Lauryl Sulphate (SLS) | 0.5 |
| Sodium Lauryl Phosphate (SLP) | 1.50 |
| Triclosan | 0.30 |
| NaF | 0.243 |
| Water | 55.857 |
| Flavor Oil | 1.00 |
| NaOH (25% Sol'n) | 0.5 |
| **TOTAL** | **100.00** |

The antibacterial activity measured as the antiplaque activity of Compositions A-F, were assessed using a chemostat plaque system of the type disclosed in the American Journal of Dentistry, Vol. 3, Special Issue, September 1990, pages S8-S9. Hydroxyapatite disks were fixed in flow cells connected to a chemostat containing a culture of bacterial growth media having a mixed culture of five species of oral microorganisms (A. viscosus, S. mutans, S. sanguis, V. parula, and F. nucleatum) associated with human plaque. The culture was pumped through the flow cells at a rate of about 1 ml/minute for 48 hours to grow plaque on the SCHAP disks.

The hydroxyapatite disks were prepared in the following manner:

Hydroxyapatite (HAP) is washed extensively with distilled water, collected by vacuum filtration, and dried overnight at 37°C. The dried HAP is ground into a powder with a mortar and pestle and 150 milligrams (mgs) of the powder is placed into a chamber of a KBr pellet die (Barnes Analytical, Stanford, Conn.). The HAP powder is compressed for 6 minutes at 10,000 pound in a Carver Laboratory press to prepare 13 mm diameter disks which are sintered for 4 hours at 800, in a Thermolyne furnace. Parafilm stimulated whole saliva is collected into an ice-chilled glass beaker and the saliva is clarified by centrifugation at 15,000 x g (times gravity) for 15 minutes at 4°C . Sterilization of the clarified-saliva is done at 4°C with stirring by irradiation of the sample with UV light for 1.0 hour.

Each sintered HAP disk is hydrated with sterile water in a polyethylene test tube. The water is then removed and replaced with 2 milliliters (ml) of saliva. A salivary pellicle is formed by incubating the disk overnight at 37°C with continuous shaking in a water bath to form a saliva coated disk (SCHAP).

To evaluate the antiplaque efficacy of Compositions A-F, each liquid dentifrice was pumped for 30 seconds, at the rate of about 1 ml/minute, through the flow cells containing the SCHAP disks on which the plaque was being grown. A total of four such treatments of the SCHAP disks were given at 12 hour intervals, during a 48 hour plaque growth period. Thereafter, the bacterial plaque grown on the SCHAP disks was removed by immersion of each disk in 2 ml of 0.1 N NaOH solution, in a water bath at 37°C, with gentle shaking for 15 minutes. The disks were removed and the NaOH solution was sonicated to disperse the plaque. The resultant plaque concentration is a function of the turbidity or optical density (O.D.) of the sample: wherein, the higher the O.D., the higher the plaque growth. The O.D. was determined by measuring the absorbence at 610 nm in a spectrophotometer - the results for Compositions A-F are presented in Table II below.

**TABLE II**

| **Plaque Growth As Measured with Chemostat Plaque System** | | | |
|---|---|---|---|
| ***Composition*** | ***SLP Content (% by Weight)*** | ***SLS Content (% by Weight)*** | ***Plaque Growth (Spectrometric O.D.)*** |
| A | 1.0 | 0.5 | 0.272 |
| B | 0.75 | 0.6 | 0.202 |
| C | 0.75 | 0.75 | 0.284 |
| D | 0.5 | 1.0 | 0.2605 |
| E | 1.5 | 0.0 | 0.3175 |
| F | 0.0 | 1.5 | 0.2943 |

The data recorded in Table II shows that for liquid dentifrice formulations having SLP to SLS ratios of from 2:1 to 1:2, respectively, there is a significant reduction in plaque growth within the chemostat, i.e. Compositions A-D as compared to compositions in which this ratio was not present, Compositions E-F.

### Example II

Using methodology disclosed in the International Dental Journal, Vol. 44. Issue No. 1, Supplement 1 (1994), by Gaffar et al., a one cell, blind, single exposure human clinical study was conducted with 5 human subjects to evaluate the effect in vivo of a paste dentifrice, Composition J, of the subject invention (comparable to Composition D of Example I regarding SLP and SLS content). The formulation of paste dentifrice J is presented in Table III. below.

The criteria for the human subjects was an age range of from 18-65, possessing a minimum of 20 uncrowned teeth, and no presence of advanced periodontal disease, extensive untreated dental caries, orthodontic appliances or other diseases of hard or soft oral tissues. Further, any subject using systemic or topical antibiotic drugs or oral antimicrobial mouthrinses or other products containing antimicrobial agents within the period of two weeks prior to the study was excluded.

The subjects were tested at about 8 a.m. without having eaten or used toothpaste or mouthwash that morning. Prior to any brushing with any dentifrice, a baseline plaque sample was collected from each subject by the dental hygienist, the sample being taken from lingual surfaces of mandibular second molars and buccal surfaces of maxillary canines. The subjects brushed in their usual manner with 1.5 g of their assigned, blind dentifrice for 45 seconds and then rinsed with water. Subsequent to the brushing the subjects were allowed to eat or drink as desired. Plaque samples where collected 6 hours after brushing for assessment of the plaque viability.

The plaque samples were placed on microscope slides and then treated (within 5 minutes of collection) with 14 ml of a solution of two fluorescent components (ethidium homodimer and 5 chloromethylfluorescein diacetate (available from Molecular Probes Incorporated, Eugene, OR) for 15 minutes at room temperature. After such incubation the slides were tipped slightly to remove any excess dye solution and were rinsed with 100 ml of phosphate buffer saline solution. This treatment resulted in differential staining of live (green stained ) and dead (red stained) bacterial cells. Cover slips were added to the samples and the slides were evaluated to obtain the plaque viability score, at 200 times magnification, under fluorescent microscopy. Ten different microscope fields of each sample were scored using the criteria of Rudegren (see Rudegren, J. Johansson, M., Astrom M., *Effect of 4-day Mouth Rinse On Delmopinol as Chlorhexidine on the Viability of Plaque Bacteria*, Journal of Periodontal Dentistry (1992)). All green was 100% viable; green with some red was 75% viable; green equal to red was 50% viable; red with some green was 25% viable ; 0 green was zero viable. Ten scores were then averaged to obtain a single value for each plaque sample from a single tooth, the higher the plaque viability score, the less viable plaque observed. The data for four teeth, canines and molars for each subject were then averaged to give a single value for each subject and all subjects were averaged to obtain a result for each comparative composition tested.

The results of applying the above clinical testing protocol to the five human subjects for paste dentifrice Composition J are provided in Table IV, below.

**Table III**

| **Paste Dentifrice Composition J** | |
|---|---|
| ***Ingredients*** | ***Wt.%*** |
| Glycerine | 20.0 |
| Carageenan | 0.3 |
| NaCMC | 0.8 |
| Propylene Glycol | 0.5 |
| NaF | 0.24 |
| Na Saccharin | 0.3 |
| TiO₂ | 0.5 |
| Sorbitol | 20.0 |
| Water (Deionized) | 32.4 |
| NaOH (25% Sol'n) | 0.16 |
| Zeodent 115 | 20.0 |
| Sylodent 15 | 2.0 |
| Triclosan | 0.3 |
| Flavor Oil | 1.0 |
| SLP | 0.5 |
| SLS | 1.0 |
| **Total** | **100.0** |

**Table IV**

| **Plaque Viability as Measured In Human Clinical Study** | | |
|---|---|---|
| ***Paste Dentifrice Composition*** | ***Baseline Plaque Viability*** | ***Plaque Viability After 6 Hours*** |
| | | |
| J | 2.089 +/- 0.007 | 2.789 +/- 0.137 |

The data on plaque viability of Table IV clearly shows a distinct efficacy advantage of the combination of SLP and SLS against the formation of plaque; a 33.5% plaque reduction versus baseline being obtained.

## Claims

1. An oral composition exhibiting increased antibacterial efficacy, the composition comprising in an orally acceptable vehicle, an effective therapeutic amount of a halogenated diphenyl ether or phenolic antibacterial compound, a monoalkyl phosphate compound and an anionic surfactant, the monoalkyl phosphate compound being present in the composition as the sole alkyl phosphate compound and the weight ratio of the monoalkyl phosphate to the anionic surfactant being in the range of 2:1 to 1:2.

2. The composition of claim 1, wherein the anionic surfactant is sodium lauryl sulfate.

3. The composition of claim 1, wherein the oral composition is a paste dentifrice.

4. The composition of claim 1, wherein the antibacterial agent is incorporated in the composition at a concentration of 0.05 to 2.0% by weight.

5. The composition of claim 1, wherein the antibacterial agent is triclosan.

6. The composition of claim 1, wherein the monoalkyl phosphate compound is incorporated in the composition at a concentration of 0.1 to 5% by weight.

7. The composition of claim 1, wherein the monoalkyl phosphate compound has the formula 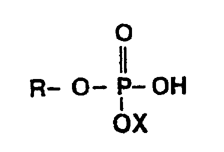 where R is an alkyl or alkenyl group having 6 to 18 carbon atoms and X is hydrogen sodium, potassium or ammonium.

8. The composition of claim 1, wherein the monoalkyl phosphate compound is sodium lauryl phosphate.

9. The composition of claim 1, wherein the monoalkyl phosphate compound is monolauryl phosphate.

10. The composition of claim 1, wherein the anionic surfactant is incorporated at a concentration of 0.1 to 5.0% by weight.

11. The composition of claim 1, wherein the anionic surfactant is sodium lauryl sulfate incorporated in the composition at a concentration of from 0.2 to 3% by weight.

12. Use of an oral composition exhibiting increased antibacterial efficacy, the composition comprising in an orally acceptable vehicle, an effective therapeutic amount of a halogenated diphenyl ether or phenolic antibacterial compound, a monoalkyl phosphate compound and an anionic surfactant, the monoalkyl phosphate compound being present in the composition as the sole alkyl phosphate compound and the weight ratio of the monoalkyl phosphate to the anionic surfactant being in the range of 2:1 to 1:2 for the manufacture of a medicament for the treatment and prevention of bacterial plaque accumulation on teeth.

13. The use of claim 12, wherein the anionic surfactant is sodium lauryl sulfate.

14. The use of claim 12, wherein the oral composition is a paste dentifrice.

15. The use of claim 12, wherein the antibacterial compound is incorporated in the composition at a concentration of 0.05 to 2.0% by weight.

16. The use of claim 12, wherein the antibacterial compound is triclosan.

17. The use of claim 12, wherein the monoalkyl phosphate compound is incorporated in the composition at a concentration of 0.1 to 5% by weight.

18. The use of claim 12, wherein the monoalkyl phosphate compound has the formula 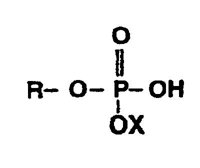 where R is an alkyl or alkenyl group having 6 to 18 carbon atoms and X is hydrogen sodium, potassium or ammonium.

19. The use of claim 12, wherein the monoalkyl phosphate compound is sodium lauryl phosphate.

20. The use of claim 12, wherein the monoalkyl phosphate compound is monolauryl phosphate.

21. The use of claim 12, wherein the anionic surfactant is incorporated at a concentration of 0.1 to 5.0% by weight.

22. The use of claim 12, wherein the anionic surfactant is sodium lauryl sulfate incorporated in the composition at a concentration of from 0.2 to 3% by weight.

## Patentansprüche

1. Orale Zusammensetzung mit verbesserter antibakterieller Wirksamkeit, die eine therapeutisch wirksame Menge halogenierte Diphenylether- oder phenolische antibakterielle Verbindung, Monoalkylphosphatverbindung und anionisches Tensid in einem oral akzeptablen Träger umfaßt, wobei die in der Zusammensetzung vorhandene Monoalkylphosphatverbindung die einzige Aklylphosphatverbindung ist und das Gewichtsverhältnis von Monoalkylphosphat zu anionischem Tensid im Bereich von 2:1 bis 1:2 liegt.

2. Zusammensetzung nach Anspruch 1, in der das anionische Tensid Natriumlaurylsulfat ist.

3. Zusammensetzung nach Anspruch 1, in der die orale Zusammensetzung eine Zahnpasta ist.

4. Zusammensetzung nach Anspruch 1, in der das antibakterielle Mittel in der Zusammensetzung in einer Konzentration von 0,05 bis 2,0 Gew.-% vorliegt.

5. Zusammensetzung nach Anspruch 1, in der das antibakterielle Mittel Triclosan ist.

6. Zusammensetzung nach Anspruch 1, in der die Monoalkylphosphatverbindung in der Zusammensetzung in einer Konzentration von 0,1 bis 5 Gew.-% vorliegt.

7. Zusammensetzung nach Anspruch 1, in der die Monoalkylphosphatverbindung die Formel 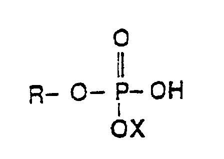 besitzt, wobei R eine Alkyl- oder Alkenylgruppe mit 6 bis 18 Kohlenstoffatomen und X Wasserstoff, Natrium, Kalium oder Ammonium ist.

8. Zusammensetzung nach Anspruch 1, in der die Monoalkylphosphatverbindung Natriumlaurylphosphat ist.

9. Zusammensetzung nach Anspruch 1, in der die Monoalkylphosphatverbindung Monolaurylphosphat ist.

10. Zusammensetzung nach Anspruch 1, in der das anionische Tensid in einer Konzentration von 0,1 bis 5,0 Gew.-% vorliegt.

11. Zusammensetzung nach Anspruch 1, in der das anionische Tensid Natriumlaurylsulfat ist und in einer Konzentration von 0,2 bis 3 Gew.-% in der Zusammensetzung vorliegt.

12. Verwendung einer oralen Zusammensetzung mit verbesserter antibakterieller Wirksamkeit, die eine therapeutisch wirksame Menge halogenierte Diphenylether- oder phenolische antibakteriellen Verbindung, Monoalkylphosphatverbindung und ein anionisches Tensid in einem oral akzeptablen Träger umfaßt, wobei die in der Zusammensetzung vorhandene Monoalkylphosphatverbindung die einzige Aklylphosphatverbindung ist und das Gewichtsverhältnis von Monoalkylphosphat zu anionischem Tensid im Bereich von 2:1 bis 1:2 liegt, für die Herstellung eines Medikaments zur Behandlung und Vorbeugung von bakteriellen Plaqueansammlungen auf Zähnen.

13. Verwendung nach Anspruch 12, wobei das anionische Tensid Natriumlaurylsulfat ist.

14. Verwendung nach Anspruch 12, wobei die orale Zusammensetzung eine Zahnpasta ist.

15. Verwendung nach Anspruch 12, wobei die antibakterielle Verbindung in einer Konzentration von 0,05 bis 2,0 Gew.-% in der Zusammensetzung vorliegt.

16. Verwendung nach Anspruch 12, wobei die antibakterielle Verbindung Triclosan ist.

17. Verwendung nach Anspruch 12, wobei die Monoalkylphosphatverbindung in der Zusammensetzung in einer Konzentration von 0,1 bis 5 Gew.-% vorliegt.

18. Verwendung nach Anspruch 12, wobei die Monoalkylphosphatkomponente die Formel 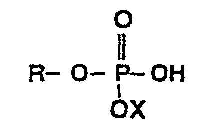 besitzt, wobei R eine Alkyl- oder Alkenylgruppe mit 6 bis 18 Kohlenstoffatomen und X Wasserstoff, Natrium, Kalium oder Ammonium ist.

19. Verwendung nach Anspruch 12, wobei die Monoalkylphosphatverbindung Natriumlaurylphosphat ist.

20. Verwendung nach Anspruch 12, wobei die Monoalkylphosphatverbindung Monolaurylphosphat ist.

21. Verwendung nach Anspruch 12, wobei das anionische Tensid in einer Konzentration von 0,1 bis 5,0 Gew.-% vorliegt.

22. Verwendung nach Anspruch 12, wobei das anionische Tensid Natriumlaurylsulfat ist und in einer Konzentration von 0,2 bis 3 Gew.-% in der Zusammensetzung vorliegt.

## Revendications

1. Composition à usage buccal présentant une efficacité antibactérienne accrue, la composition comprenant, dans un véhicule acceptable pour l'usage buccal, une quantité thérapeutique efficace d'un éther de diphényle halogéné ou d'un composé phénolique antibactérien, un phosphate de monoalkyle, et un agent tensio-actif anionique, le phosphate de monoalkyle étant présent dans la composition comme seul phosphate d'alkyle et le rapport en poids du phosphate de monoalkyle à l'agent tensio-actif anionique étant compris dans l'intervalle de 2:1 à 1:2.

2. Composition selon la revendication 1, dans laquelle l'agent tensio-actif anionique est le laurylsulfate de sodium.

3. Composition selon la revendication 1, dans laquelle la composition à usage buccal est une pâte dentifrice.

4. Composition selon la revendication 1, dans laquelle l'agent antibactérien est incorporé dans la composition à une concentration de 0,05 à 2,0 % en poids.

5. Composition selon la revendication 1, dans laquelle l'agent antibactérien est le triclosan.

6. Composition selon la revendication 1, dans laquelle le phosphate de monoalkyle est incorporé dans la composition à une concentration de 0,1 à 5 % en poids.

7. Composition selon la revendication 1, dans laquelle le phosphate de monoalkyle répond à la formule 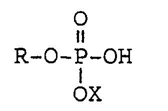 où R est un groupe alkyle ou alcényle ayant 6 à 18 atomes de carbone et X est l'hydrogène, le sodium, le potassium ou l'ammonium.

8. Composition selon la revendication 1, dans laquelle le phosphate de monoalkyle est le laurylphosphate de sodium.

9. Composition selon la revendication 1, dans laquelle le phosphate de monoalkyle est le phosphate de monolauryle.

10. Composition selon la revendication 1, dans laquelle l'agent tensio-actif anionique est incorporé à une concentration de 0,1 à 5,0 % en poids.

11. Composition selon la revendication 1, dans laquelle l'agent tensio-actif anionique est le laurylsulfate de sodium incorporé dans la composition à une concentration de 0,2 à 3 % en poids.

12. Utilisation d'une composition à usage buccal présentant une efficacité antibactérienne accrue, la composition comprenant, dans un véhicule acceptable pour l'usage buccal, une quantité thérapeutique efficace d'un éther de diphényle halogéné ou d'un composé phénolique antibactérien, un phosphate de monoalkyle et un agent tensio-actif anionique, le phosphate de monoalkyle étant présent dans la composition comme seul phosphate d'alkyle et le rapport en poids du phosphate de monoalkyle à l'agent tensio-actif anionique étant compris dans l'intervalle de 2:1 à 1:2, pour la préparation d'un médicament destiné au traitement et à la prévention de l'accumulation de plaque bactérienne sur les dents.

13. Utilisation selon la revendication 12, dans laquelle l'agent tensio-actif anionique est le laurylsulfate de sodium.

14. Utilisation selon la revendication 12, dans laquelle la composition à usage buccal est une pâte dentifrice.

15. Utilisation selon la revendication 12, dans laquelle le composé antibactérien est incorporé dans la composition à une concentration de 0,05 à 2,0 % en poids.

16. Utilisation selon la revendication 12, dans laquelle le composé antibactérien est le triclosan.

17. Utilisation selon la revendication 12, dans laquelle le phosphate de monoalkyle est incorporé dans la composition à une concentration de 0,1 à 5 % en poids.

18. Utilisation selon la revendication 12, dans laquelle le phosphate de monoalkyle répond à la formule 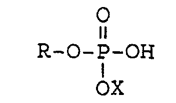 où R est un groupe alkyle ou alcényle ayant 6 à 18 atomes de carbone et X est l'hydrogène, le sodium, le potassium ou l'ammonium.

19. Utilisation selon la revendication 12, dans laquelle le phosphaté de monoalkyle est le laurylphosphate de sodium.

20. Utilisation selon la revendication 12, dans laquelle le phosphate de monoalkyle est le phosphate de monolauryle.

21. Utilisation selon la revendication 12, dans laquelle l'agent tensio-actif anionique est incorporé à une concentration de 0,1 à 5,0 % en poids.

22. Utilisation selon la revendication 12, dans laquelle l'agent tensio-actif anionique est le laurylsulfate de sodium incorporé dans la composition à une concentration de 0,2 à 3 % en poids.
